# EUROPEAN PATENT SPECIFICATION

(11) **EP 4 385 471 B1**
(45) Date of publication and mention of the grant of the patent: **20.08.2025**
(21) Application number: 22213864.6
(22) Date of filing: 15.12.2022
(51) Int. Cl.: A61F 13/00, A61F 13/02

(54) **A NEGATIVE PRESSURE WOUND THERAPY (NPWT) DRESSING**
VERBAND FÜR UNTERDRUCKWUNDTHERAPIE (NPWT)
PANSEMENT POUR THÉRAPIE DE PLAIE PAR PRESSION NÉGATIVE (NPWT)

(43) Date of publication of application: 19.06.2024
(73) Proprietor: Mölnlycke Health Care AB, 431 21 Mölndal (SE)
(72) Inventor: HOLMÉN, Malin, 412 66 GÖTEBORG (SE); BOLYOS, Elinor, 438 91 LANDVETTER (SE)
(74) Representative: Kransell & Wennborg KB

(56) References cited:
- EP-A1- 3 915 532

## Description

### TECHNICAL FIELD

The present disclosure generally relates to a negative pressure wound therapy (NPWT) dressing. It also relates to a system comprising such an NPWT dressing and to a method of manufacturing the NPWT dressing.

### BACKGROUND

Negative pressure wound therapy (NPWT) is a technique that promotes healing of e.g. surgical, acute and chronic wounds by the application of a sub-atmospheric pressure to the wound, using a negative pressure pump. Wound healing is achieved by applying a negative pressure, such as vacuum through a dressing or a cover applied onto the wound. Excess wound exudate is thereby drawn out, which increases the blood flow to the area, and promotes the formation of granulation tissue. The NPWT technique also permits less outside disturbance of the wound and may transport excess fluids away from the wound site. NPWT dressings are known from EP 3915532.

The NPWT technique has, until now, mainly been applied to a patient while in a hospital environment. However, recent product development allows the technique to be used by a patient in a home environment.

In a home environment, a portable NPWT device, which may be carried around by the patient, is generally preferred. A portable NPWT device typically comprises an absorbent dressing configured to be connected to a negative pressure source by means of tubing.

Absorbent dressings utilized in negative pressure wound therapy often comprise a spacer fabric material.

A spacer fabric material is a three-dimensional knitted fabric which comprises a top layer and a bottom layer being connected by a layer of pile filaments or threads. The pile filaments extending between the top layer and bottom layer secure that a defined distance is established between the top layer and the bottom layer.

The spacer fabric layer is resistant to compression and is configured to withstand pressures exerted on the dressing during use. After a compressive force has been exerted to the dressing, the spacer fabric material is configured to return to its original shape immediately after removal of the force. Furthermore, the spacer fabric material ensures that negative pressure can be transmitted to the wound area in both wet and dry conditions.

However, during manufacturing of the NPWT dressing, the various components of the dressing are typically cut before assembly of the dressing. When cutting through a spacer fabric layer, the interconnecting pile filaments extending between the top layer and bottom layer may lose their anchoring to the top or bottom layer and may project outwardly, i.e. laterally from the spacer fabric material.

This may create problems when the dressing is assembled with other dressing components, particularly with the backing layer, i.e. the top layer of the dressing.

If the dressing is a so-called bordered dressing, i.e. a dressing wherein the backing layer, and sometimes also the wound contact layer, extends beyond the contour of the other layers (e.g. a spacer fabric layer and an absorbent layer) to form a border portion, the outwardly projecting pile filaments may puncture and disrupt the backing layer of the dressing.

The backing layer is typically a thin polymeric film and is particularly prone to the formation of small holes. If such small holes are formed in the backing layer, air may leak into the system, resulting in an impaired or even failed therapy. Furthermore, the wear time of the dressing is shortened since a ruptured backing layer typically results in that the dressing must be discarded.

In view of the above, there is a need for improvement with respect to dressings for use in negative pressure wound therapy, particularly with respect to improving the wear time of such dressings and in preventing the backing layer from becoming disrupted such that a stable and reliable negative wound therapy is enabled.

### SUMMARY

In view of the above-mentioned problems, it is an object of the present disclosure to provide improvements with respect to dressings for NPWT applications, particularly with respect to providing an improved wear time and preventing tearing of the backing layer such that the entire NPWT system and applied therapy works in an efficient manner.

According to a first aspect, there is provided a negative pressure wound therapy (NPWT) dressing configured to be connected to a negative pressure source; the NPWT dressing comprising, from top-to-bottom, a backing layer, a liquid spreading layer, an absorbent pad comprising one or a plurality of pad-forming layers, a spacer fabric layer, and an adhesive skin contact layer; the backing layer and the adhesive skin contact layer being arranged to extend beyond the contour of the liquid spreading layer, absorbent pad and spacer fabric layer to form a border portion, wherein the spacer fabric layer comprises a top layer, a bottom layer and an interconnecting layer arranged between the top layer and the bottom layer; the interconnecting layer comprising a plurality of pile filaments extending between the top layer and the bottom layer, wherein the top layer of the spacer fabric layer is defined by peripheral edges, and wherein at least one of the liquid spreading layer and the pad-forming layer(s) of the absorbent pad is arranged to overlap the peripheral edges of the top layer of the spacer fabric layer.

The present invention is based on the realization that the backing layer may be prevented from becoming punctured by pile filaments of the interconnecting layer of the spacer fabric layer if at least one overlying layer; i.e. the liquid spreading layer or at least one of the pad-forming layers of the absorbent pad overlaps the peripheral edges of the top layer of the spacer fabric.

The top layer and the bottom layer of the spacer fabric are typically coextensive; i.e. the top layer and the bottom layer have the same surface area. Hence, the liquid spreading layer or the pad-forming layer(s) of the absorbent pad will overlap the peripheral edges of the bottom layer of the spacer fabric layer as well.

When the spacer fabric is cut into shape prior to assembly of the dressing, the pile filaments extending between the top layer and the bottom layer may lose their anchoring to the top layer and/or the bottom layer. Instead of projecting in a defined and "organized" manner between the top layer and the bottom layer, the pile filaments may project outwardly, i.e. in a lateral direction. In this regard, the backing layer, which is configured to extend beyond the edges of the spacer fabric layer, and the other layers (the liquid spreading layer and the pad-forming layer(s)), may become disrupted by the laterally extending pile filaments. The pile filaments are typically stiff and thin and may create small micro holes in the thin backing layer, particularly in the areas close to the peripheral edges of the pad-forming layers.

The overlapping arrangement of the liquid spreading layer and/or the pad-forming layers secure that the laterally projecting pile filaments become shielded such that the backing layer is protected from becoming punctured.

Accordingly, the wear time of the dressing may be enhanced since the dressing does not need to be discarded before reaching its full absorbent capacity.

Furthermore, a stable and reliable negative pressure wound therapy is achieved as failure resulting from air leakage due to a disrupted backing layer is avoided.

The dressing of the present disclosure is a so-called "bordered dressing". A bordered dressing is attached to the skin at the adhesive border portions, or at the entire surface of the adhesive skin contact layer of the dressing. For bordered dressings, detachment of the dressing may initiate at the edges of the absorbent pad. During use of the dressing, e.g. as a patient moves or bends, the backing layer is subject to tension. The backing layer typically extends obliquely from the edges of the absorbent pad, which is generally much thicker than the backing layer. This way, air pockets may form around the edges of the pad. As the backing layer is stretched, the air pockets increase, which may lead to detachment of the dressing from the skin. Accumulation of fluids in the air pockets further impairs the wear time of the dressing. The mechanical tension on the backing layer may also cause shear stresses on the adhesive skin contact layer, that in turn results in detachment.

To solve this issue, the backing layer may be pre-formed into shape prior to assembly with the other dressing components, i.e. the liquid spreading layer, the absorbent pad, the spacer fabric layer and the adhesive skin contact layer.

Hence, in embodiments, the backing layer has a central portion and an edge portion surrounding the central portion, wherein the central portion has a pre-formed shape complementary to the shape of at least the absorbent pad, and wherein the edge portion forms part of the border portion.

By pre-forming the backing layer into a shape that matches at least the absorbent pad, the backing layer will conform to the edges of these dressing layers. Accordingly, the backing layer is arranged in close proximity of these edges such that essentially no, or only minor gaps are formed around the periphery of the pad-forming layers. The tension formed near the edges of the absorbent pad and liquid spreading layer is thus considerably reduced. Accordingly, the risk of detachment from the skin is reduced as well as the risk for pressure being built up around the edges of the pad.

While this configuration allows the wear time of the dressing to be improved, it also enhances the problem outlined hereinbefore, i.e. that the backing layer may become punctured and disrupted by the outwardly projecting pile filaments. This problem is particularly enhanced if the backing layer is arranged in close proximity of the spacer fabric.

The provision of an overlap by the liquid spreading layer and/or the pad-forming layer(s) with respect to the spacer fabric layer secures that this problem is avoided.

In exemplary embodiments, the spacer fabric layer has a thickness, t1, and wherein at least one of the liquid spreading layer and the pad-forming layer(s) of the absorbent pad is arranged to overlap the peripheral edges by a distance, d1, wherein the distance d1 corresponds to 0.7 to 4.0t1, preferably 1.0 to 2.0t1.

A distance, d1, within these ranges secures that the problem of disrupting the backing layer is overcome or at least significantly reduced. Furthermore, a distance within these ranges secures that an efficient transmission of negative pressure is accomplished between the negative pressure source and the wound site. If the overlap is too large, the transmission of negative pressure and the applied therapy may be reduced.

In exemplary embodiments, the NPWT dressing further comprises a coupling member configured to connect the dressing to a negative pressure source, wherein the backing layer and at least a portion of the absorbent pad comprises an opening underneath the coupling member; the liquid spreading layer being void of an opening.

An opening is typically provided in the absorbent pad to facilitate transmission of negative pressure. The fact that the liquid spreading layer does not contain any opening prevents gelling particles and undesired larger particulate from entering the coupling member and the tubing connected thereto.

In exemplary embodiments, the liquid spreading layer is a continuous layer extending across the entire surface area of the absorbent pad.

The liquid spreading layer improves the spreading of liquid across a large surface area such that moisture can evaporate through the backing layer in a more efficient manner.

When the dressing of the present disclosure is utilized in an NPWT system comprising a remote fluid collection means, e.g. a canister, a portion of the wound exudate will be transported away from the dressing to the canister (typically by means of a tubing connecting the dressing with the canister and the negative pressure source). In such cases, the liquid spreading layer secures that any potential back-flow of exudate from the canister to the wound site is spread out across a large surface rather than flowing back towards the wound site in one spot. Accordingly, the wound site is kept relatively dry.

In exemplary embodiments, the liquid spreading layer is a nonwoven layer.

The nonwoven liquid spreading layer aids in driving the fluid away from the wound site and from the wound pad, while at the same time securing that the maximum capacity of the absorbent dressing can be utilized.

Furthermore, the nonwoven layer imparts an appropriately balanced rigidity to the layer and to the dressing as such. More particularly, the nonwoven layer protects the overlying backing layer from becoming punctured.

In exemplary embodiments, the absorbent pad comprises a first liquid distribution layer, a superabsorbent layer and a second liquid distribution layer, wherein the superabsorbent layer is arranged between the first and the second liquid distribution layers, and wherein the first or the second liquid distribution layer is a nonwoven layer.

The first liquid distribution layer is configured to absorb and distribute liquid flowing from the wound site. The first liquid distribution layer may distribute and spread the wound exudate evenly and over a large surface area such that it can be absorbed by the superabsorbent layer. The second liquid distribution layer distributes the exudate from the superabsorbent layer such that the exudate is spread over a large area before being evaporated from the backing layer.

In embodiments where the NPWT system also comprises a remote fluid collection means, such as a canister, the configuration of the absorbent pad secures that a balanced distribution of liquid is enabled within the dressing, while also enabling transport of wound exudate to the remote fluid collection means by means of the tubing.

The absorbent pad along with the liquid distribution layer overlying the absorbent pad is configured to optimize the distribution of wound exudate within the dressing. Where the NPWT dressing is connected to a remote fluid collection means, the configuration of the absorbent pad secures removal of a substantial amount of exudate to the remotely arranged fluid collection means in a controlled manner. The absorbent pad is thus designed to achieve an appropriate liquid distribution balance between the dressing and the remote fluid collection means, which both may serve as fluid "compartments" for holding and storing liquids.

In exemplary embodiments, both the liquid spreading layer and the nonwoven layer of the absorbent pad are arranged to overlap the peripheral edges of the spacer fabric layer.

This arrangement allows for an enhanced protection against puncture and rupture of the backing layer.

In exemplary embodiments, the liquid spreading layer and each pad-forming layer of the absorbent pad are arranged to overlap the peripheral edges of the top layer of the spacer fabric layer.

This arrangement allows for an improved shielding of the laterally extending pile filaments of the interconnecting layer of the spacer fabric. Accordingly, the protection against backing layer rupture is significantly enhanced.

In exemplary embodiments, the combined thickness of the liquid spreading layer and the absorbent pad is larger than the thickness, t1, of the spacer fabric layer.

The combined thickness of the liquid spreading layer and the absorbent pad is significantly larger than the spacer fabric layer and its interconnecting pile filaments.

Accordingly, a cushioning effect is achieved at the peripheral edges of the spacer fabric layer, which results in that the pile filaments will not reach the overlying backing layer.

In exemplary embodiments, the dressing comprises a tubing, wherein a first distal end of the tubing is connected to the coupling member and a second distal end of the tubing is configured to be connected to a negative pressure source, wherein the tubing comprises a fluid conduit configured to remove fluid from the dressing and an air conduit configured to supply air to the fluid conduit and/or the dressing.

A small and controlled inflow of air may be beneficial to more efficiently draw fluid from the wound site and transport the fluid to a remotely arranged fluid collection means, e.g. the canister. The introduction of air may resolve potential exudate blockages or liquid columns formed in the tubing.

According to another aspect, there is provided a negative pressure wound therapy (NPWT) system comprising:
- a negative pressure wound therapy (NPWT) dressing as described hereinbefore,
- a negative pressure source, and
- a remote fluid collection means fluidly connected to the negative pressure source and to the dressing.

Hence, the NPWT system comprises two exudate storage means; i.e. the absorbent pad as well as a remote fluid collection means, e.g. a canister. This is beneficial to avoid exudate saturation within the dressing, which could ultimately lead to loss of adhesion to the skin. The provision of a remote fluid collection means may thus enhance the wear time of the dressing since two separate fluid handling systems are comprised in the NPWT system.

In exemplary embodiments, the remote fluid collection means is a canister and wherein the canister and the negative pressure source are arranged within the same device.

The device is typically portable and may comprise a housing, in which the negative pressure source is arranged. The canister may be detachably connected to the housing.

The detachable configuration allows the user or caregiver to remove the canister and empty the collected liquid, and subsequently re-attach the canister to the negative pressure source again.

In exemplary embodiments, the system may comprise means to supply air to the dressing at a rate of from 2 to 7 ml/min during operation.

A small and controlled inflow of air may be beneficial to more efficiently draw fluid from the wound site and transport the fluid to the remotely arranged fluid collection means, e.g. the canister. It may also be beneficial to avoid potential obstructions in the tubing connecting the dressing with the negative pressure source and the remote fluid collection means and to secure that the desired pressure level is transmitted to the wound site. In negative pressure wound therapy systems, there is typically a static pressure difference introduced by gravity between the pressure inside the canister and the pressure at the wound site. This is due to the height difference between the canister and the wound site. A change in the static pressure may affect the ability to provide the correct level of negative pressure at the wound site. The provision of a small air flow or air leakage resolves these problems. Furthermore, if too much air is introduced, this may negatively impact the stability of the system, and the pump must typically be activated on a higher frequency.

According to yet another aspect, there is provided a method for manufacturing a negative pressure wound therapy (NPWT) dressing, wherein the method comprises:
a) providing a backing layer,
b) arranging a liquid spreading layer in contact with the backing layer,
c) arranging an absorbent pad comprising one or a plurality of pad-forming layers in contact with the liquid spreading layer,
d) arranging a spacer fabric layer in contact with the absorbent pad, wherein the spacer fabric layer comprises a top layer, a bottom layer and an interconnecting layer arranged between the top layer and the bottom layer; the interconnecting layer comprising a plurality of pile filaments extending between the top layer and the bottom layer, wherein the top layer of the spacer fabric layer is defined by peripheral edges,
e) arranging an adhesive skin contact layer in contact with the spacer fabric layer,

wherein the adhesive skin contact layer and the backing layer are arranged to extend beyond the contour of the liquid spreading layer, the absorbent pad, and the spacer fabric layer to form a border portion, and
wherein at least one of the liquid spreading layer and the pad-forming layer(s) of the absorbent pad is arranged to overlap the peripheral edges of the top layer of the spacer fabric layer.

**In** exemplary embodiments, the backing layer may be pre-formed to a shape complementary to the shape of at least the absorbent pad prior to or simultaneously with the step b) of arranging the liquid spreading layer in contact with the backing layer.

The backing layer will thus conform to the edges of the absorbent pad, thereby reducing the tension on the backing layer, and preventing gaps from forming around the edges of the absorbent pad.

**In** addition to preventing detachment from the skin, pre-forming of the backing layer also prevents the formation of folds in the backing layer, when the dressing is assembled with the other dressing layers.

In exemplary embodiments, the backing layer is pre-formed by:
a) contacting the backing layer with a molding tool having a shape complementary to the shape of at least the absorbent pad, and
b) applying vacuum to the backing layer.

The application of vacuum allows the backing layer to be stretched and drawn into the molding tool, and thereby adopting a shape corresponding to the molding tool. More particularly, the central portion of the backing layer will adopt a shape that corresponds to the molding tool; i.e. the shape of at least the absorbent pad. Typically, the molding tool has a shape complementary to the absorbent pad and the liquid spreading layer.

This improves the ability of the backing layer to conform to the peripheral edges of the absorbent pad.

In exemplary embodiments, the steps b)-e) of arranging the liquid spreading layer, the absorbent pad, and the spacer fabric layer are performed simultaneously with contacting the backing layer with a molding tool having a shape complementary to the shape of at least the absorbent pad, and preferably while simultaneously applying vacuum to the backing layer.

Accordingly, the pre-formed shape of the backing layer is affixed, and the backing layer will conform to the peripheral edges of the absorbent pad.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 illustrates a negative pressure wound therapy (NPWT) dressing according to an exemplary embodiment of the present disclosure.
Figure 2 illustrates a split view of the NPWT dressing of figure 1.
Figure 3a illustrates a cross-sectional view of the NPWT dressing of figure 1.
Figures 3b and 3c illustrate zoomed-in views of the dressing of figure 3a.
Figure 4a illustrates an NPWT dressing having a similar construction as the NPWT dressing of figure 3a, but wherein the spacer fabric layer has the same outer dimensions as the absorbent pad and the liquid spreading layer.
Figures 4b and 4c illustrate zoomed-in views of the dressing of figure 4a.
Figure 5 conceptually illustrates a negative pressure wound therapy (NPWT) system according to an exemplary embodiment of the present disclosure.
Figure 6 illustrates the test equipment utilized in Example 2.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

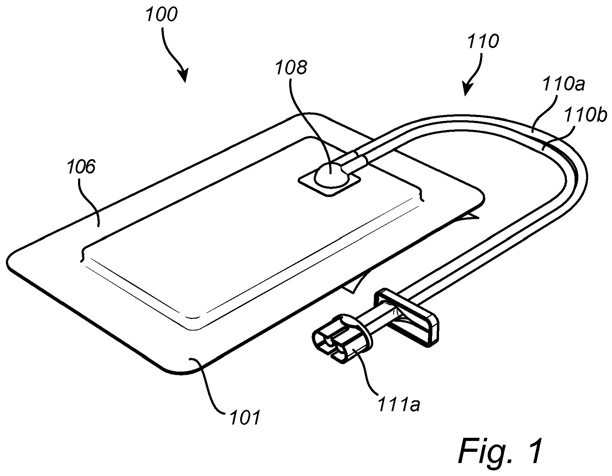

Figures 1-3 illustrates an NPWT dressing according to an exemplary embodiment of the present disclosure.

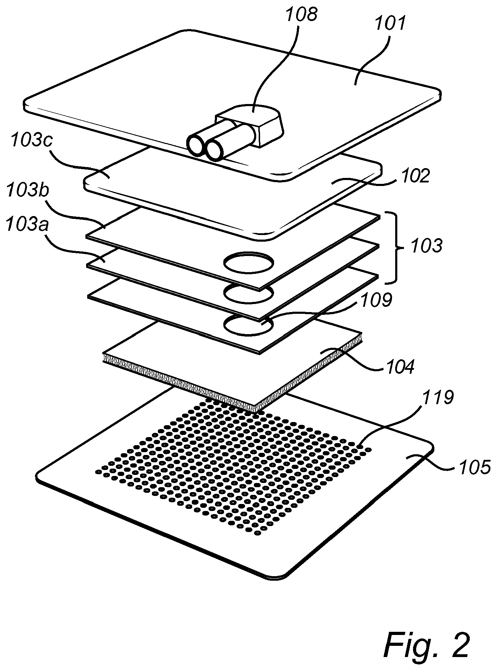

As best illustrated in figures 2, 3a, and 4a-b the NPWT dressing 100 comprises, from top-to-bottom, a backing layer 101, a liquid spreading layer 102, an absorbent pad 103 comprising one or a plurality of pad-forming layers 103a-c, a spacer fabric layer 104, and an adhesive skin contact layer 105; the backing layer 101 and the adhesive skin contact layer 105 being arranged to extend beyond the contour of the liquid spreading layer 102, the absorbent pad 103 and the spacer fabric layer 104 to form a border portion 106, wherein the spacer fabric layer 104 comprises a top layer 104a, a bottom layer 104b and an interconnecting layer 104c arranged between the top layer 104a and the bottom layer 104b; the interconnecting layer 104c comprising a plurality of pile filaments 107 extending between the top layer 104a and the bottom layer 104b, wherein the top layer 104a of the spacer fabric layer 104 is defined by peripheral edges, and wherein at least one of the liquid spreading layer 102 and the one or more pad-forming layer(s) 103a-c of the absorbent pad 103 is arranged to overlap the peripheral edges of the top layer of the spacer fabric layer 104.

As used herein, the term "negative pressure wound therapy dressing" refers to a dressing for use in negative pressure wound therapy. In the context of the present disclosure, "negative pressure wound therapy" refers to a therapy utilizing a source of negative pressure (e.g. a vacuum pump) to remove excess fluid from a wound. The wound may be an open wound or it may be a closed wound; i.e. a surgically closed incision, and the term therefore also encompasses "topical negative pressure (TNP) therapy" applications, which is a term often used in the context of closed incisions.

The "spacer fabric layer" is a three-dimensional knitted fabric which comprises a top layer and a bottom layer being connected by an interconnecting layer of pile filaments. The pile filaments extend between the top layer and bottom layer and secure that a defined distance is established between the layers. The top layer of the spacer fabric layer is arranged distal from the skin of the patient in use. The bottom layer of the spacer fabric layer is arranged proximal to the skin of the patient in use.

The spacer fabric layer is not limited to a specific material. In exemplary embodiments, the spacer fabric layer comprises polyester. The pile filaments of the interconnecting layer may be polyester monofilament fibers.

The "peripheral edges" of the top layer of the spacer fabric layer may represent a pair of lateral edges and a pair of longitudinal edges if the spacer fabric layer has a square or rectangular shape. The spacer fabric layer (and the dressing) is not limited to a square or rectangular shape, but may be circular, oval or any other conceivable shape. The peripheral edges define the boundaries of the spacer fabric layer.

The liquid spreading layer or one or a plurality of the pad-forming layers overlap the peripheral edges of the top layer of the spacer fabric layer. The top layer is typically co-extensive with the bottom layer. Accordingly, the liquid spreading layer or one or a plurality of the pad-forming layers also overlap the peripheral edges of the bottom layer of the spacer fabric layer.

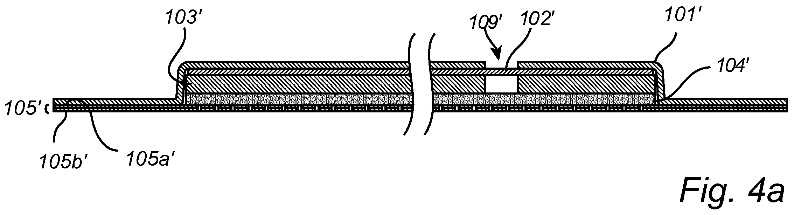

Figures 4a-c illustrate an NPWT dressing with a similar construction as the dressing of the present disclosure, but wherein the spacer fabric layer 104' has the same outer dimensions as the overlying absorbent pad 103' and liquid spreading layer 102'.

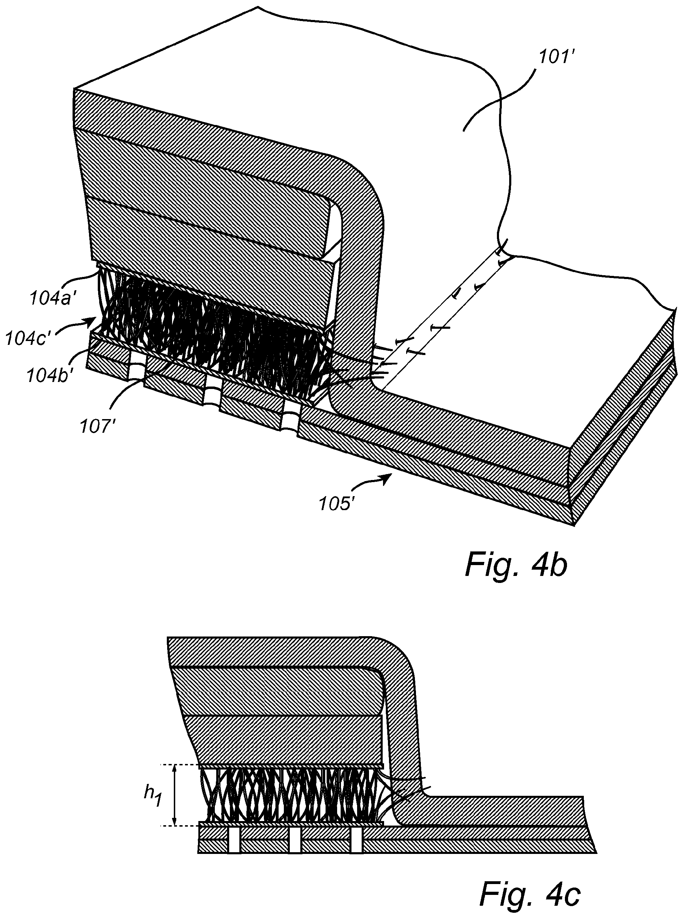

As best illustrated in the zoomed-in view in figures 4b and 4c, some of the pile filaments 107' of the spacer fabric layer 104' project laterally towards the backing layer 101', which overlies and overlaps the peripheral edges of the spacer fabric layer 104' (and the absorbent pad 103' and liquid spreading layer 102').

Accordingly, the pile filaments 107' have lost their attachment to the top layer 104a' and/or the bottom layer 104b' of the spacer fabric layer. This may for instance occur when the spacer fabric layer is cut into shape during assembly of the dressing components.

As a result, and as seen in figures 4b and 4c, the laterally projecting pile filaments 107', which are typically very thin and sharp, puncture the backing layer 101 and project through this layer, thereby yielding small holes, typically micro holes. When this occurs, the NPWT dressing must typically be discarded. The negative pressure source, i.e. the vacuum pump must work much harder and the batteries will be worn out. Accordingly, the applied negative pressure wound therapy must typically be discontinued since air will leak into the system. The wear time of the dressing is thus considerably reduced.

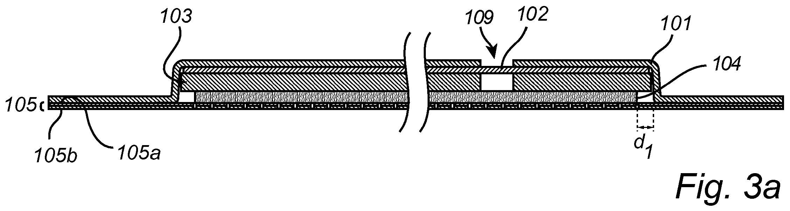

As best illustrated in figures 3a-c, the above-described problem is overcome, or at least significantly reduced by arranging at least one of the liquid spreading layer 102 and one or more of the pad-forming layer(s) 103a-c of the absorbent pad 103 such that it overlaps the peripheral edges of the top layer 104a of the spacer fabric layer 104.

Since the top layer 104a is coextensive with the bottom layer 104b of the spacer fabric layer 104, the liquid spreading layer 102 and/or one or more of the pad-forming layer(s) 103a-c of the absorbent pad 103 typically also overlap the bottom layer 104b of the spacer fabric layer 104, as mentioned hereinbefore.

With this construction, the pile filaments 107 are prevented from puncturing the backing layer 101.

As can be seen in figures 3a-3c, the backing layer 101 is arranged in close proximity with the peripheral edges of the liquid spreading layer 102 and the absorbent pad 103, and conforms to the edges of these layers such that essentially no gaps are present.

The backing layer 101 may have a central portion and an edge portion surrounding the central portion, wherein the central portion has a pre-formed shape complementary to the shape of at least the absorbent pad 103, and wherein the edge portion of the backing layer 101 forms part of the border portion 106 of the dressing.

Typically, the central portion has a pre-formed shape complementary to the shape of the liquid spreading layer 102 and the absorbent pad 103.

As used herein, the term "complementary to the shape of the liquid spreading layer and the absorbent pad" means that the central portion of the backing layer has substantially the same outer dimensions, i.e. width and length, as the outer dimensions of the liquid spreading layer and the absorbent pad. The depth of the backing layer in the central portion corresponds to the thickness of the liquid spreading layer and the absorbent pad, and preferably also the thickness of the spacer fabric layer.

This arrangement prevents gaps from being formed in the areas surrounding the peripheral edges of the absorbent pad and the liquid spreading layer.

Such gaps may give rise to detachment of the dressing from the skin, and thereby impair the wear time of the dressing. However, the close adherence of the backing layer to these dressing layers also enhances the problem of backing layer puncture.

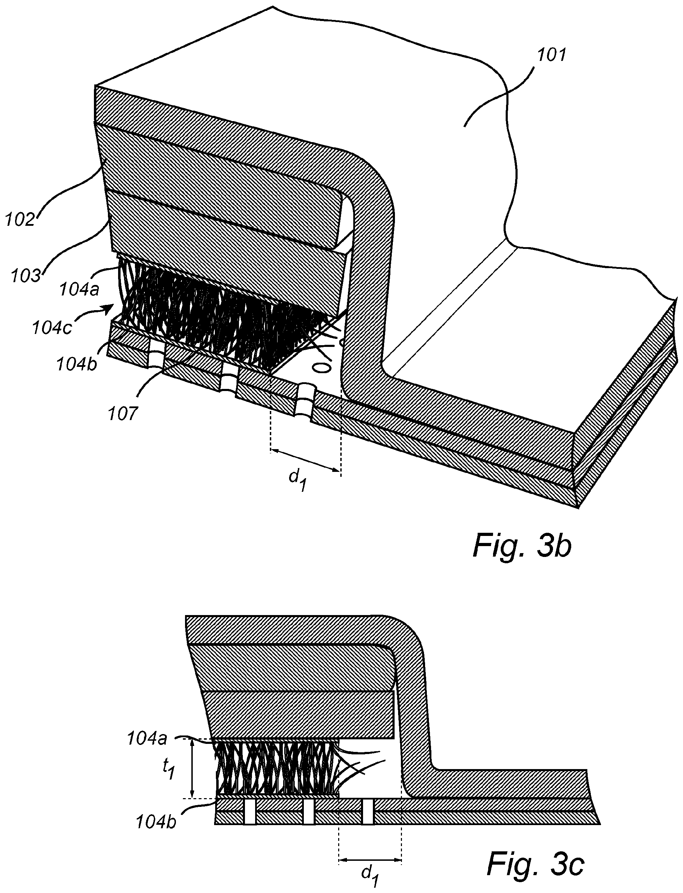

As illustrated in figure 3b, the spacer fabric layer may have a thickness, t1, and wherein at least one of the liquid spreading layer and the pad-forming layer(s) 103a-c of the absorbent pad 103 may be arranged to overlap the peripheral edges by a distance, d1, wherein the distance d1 corresponds to 0.7 to 4.0t1, preferably 1.0 to 2.0t1.

The thickness, t1, is measured in dry conditions and without application of pressure.

The thickness, t1, of the spacer fabric layer corresponds to the combined thickness of the top layer, interconnecting layer, and the bottom layer.

The thickness, t1, of the spacer fabric layer may be from 1.5 to 4 mm, preferably from 2 to 3 mm. The basis weight of the spacer fabric layer may be from 150 to 500 gsm, e.g. from 200 to 350 gsm.

The interconnecting layer of pile filaments may have a fineness of 200 to 500 denier, e.g. from 250 to 350 denier.

A distance, d1, between 0.7 to 4.0t1, preferably 1.0 to 2.0t1, prevents the backing layer from becoming ruptured by the spacer fabric pile filaments, and also secures that a significant portion of the spacer fabric layer is utilized for transmission of negative pressure. If the overlap is too large, the transmission of negative pressure and the efficiency of the applied therapy may be reduced. The overlap is typically larger than the length of the pile filaments of the interconnecting layer 104c of the spacer fabric layer 104.

As best illustrated in figures 2 and 3, the NPWT dressing may further comprise a coupling member 108 configured to connect the dressing to a negative pressure source, wherein the backing layer 101 and at least a portion of the absorbent pad 103 comprises an opening 109 underneath the coupling member 108; the liquid spreading layer 102 being void of an opening.

The opening 109 ensures fluid communication between the wound site and the remotely arranged fluid collection means. It also enables transmission of negative pressure to the wound site. The coupling member 108 overlies the opening 109 in the backing layer. In figure 2, the absorbent pad 103 comprises three layers, each of which comprises an opening. It is however also conceivable that an opening is provided in only one or in two layers of the absorbent pad 103.

The coupling member is configured to be connected to a tubing 110 which connects the NPWT dressing to a negative pressure source, e.g. a vacuum pump.

The fact that the liquid spreading layer 102 does not contain any opening prevents gelling particles and undesired larger particulate from entering the tubing 110 of the dressing 100.

It also prevents potential back-flow of exudate in embodiments where the NPWT system comprises a remote fluid collection means, e.g. a canister.

Back-flow of exudate, i.e. exudate flowing from the tubing towards the dressing, may occur if the person wearing the dressing disconnects the dressing from the negative pressure source and the canister. For example, the patient may disconnect the NPWT dressing if he/she is to take a shower or change clothes. The liquid spreading layer 102 secures that such back-flow of exudate is spread out rather than flowing back towards the wound site in one spot. This way, the wound site can be kept relatively dry.

As illustrated in figures 2 and 3, the liquid spreading layer 102 may be configured to extend across the entire surface area of the absorbent pad 103.

The liquid spreading layer 102 preferably extends across and overlies the opening 109 in the absorbent pad 103.

In exemplary embodiments, the liquid spreading layer 102 and the absorbent pad 103 have the same outer dimensions.

The liquid spreading layer 102 is configured to improve the spreading of wound exudate and to create a larger surface area from which moisture can evaporate through the backing layer 101.

The liquid spreading layer 102 is preferably a hydrophilic and porous layer. The liquid spreading layer 102 may be a fibrous material.

In embodiments the liquid spreading layer 102 is a nonwoven layer.

A nonwoven layer yields rigidity to the dressing and, in cases where the nonwoven layer is arranged to overlap the peripheral edges of the top layer of the spacer fabric layer, the nonwoven layer enhances the protection of the backing layer and prevents puncture or rupture of the backing layer.

Furthermore, a nonwoven liquid spreading layer 102 has the ability to distribute fluid throughout the majority of the material and to transfer the exudate in a controlled manner to the tubing 110 connecting the dressing with a remotely arranged fluid collection means, where present. The liquid spreading layer 102 aids in driving the fluid away from the wound site and from the absorbent pad 103, while at the same time securing that the maximum capacity of the absorbent dressing is utilized.

The liquid spreading layer 102 may comprise a meltblown, spunbond or a spunlaced nonwoven. Examples of suitable polymers for use in the nonwoven are polyethylene, polyesters, polypropylene and other polyolefin homopolymers and copolymers. For example, nonwoven webs comprising thermoplastic fibers of polypropylene and polyethylene fibres or mixtures thereof may be used. The webs may have a high content of thermoplastic fibres and contain at least 50%, e.g. at least 70% thermoplastic fibres. The nonwoven may be a mixture of polyester and viscose, e.g. in a 70:30 ratio. The basis weight of the nonwoven may be in the range of from 10 to 80 g/m2, e.g. of from 20 to 50 g/m2. The liquid spreading layer may also be a spunbond- meltblown or spunbond-meltblown-spunbond (SMS) web.

The absorbent pad 103 may comprise one or a plurality of layers, wherein at least one of the layers comprises a superabsorbent layer comprising superabsorbent polymers (SAP).

A "superabsorbent polymer" or "SAP" is a polymer that can absorb up to 300 times its own weight in aqueous fluids. Superabsorbent polymers are constituted by waterswellable and water insoluble polymers capable of absorbing large quantities of fluid upon formation of a hydrogel. The superabsorbent polymers for use in accordance with the present disclosure may be inorganic or organic crosslinked hydrophilic polymers, such as polyvinyl alcohols, polyethylene oxides, crosslinked polyacrylates and the like. Typically, the superabsorbent (SAP) comprise sodium acrylate. The SAP material may be in the form of particles, fibers, flakes or similar. Preferably, the SAP material is in the form of superabsorbent polymer (SAP) particles. The size of the superabsorbent particles may be in the range of from 45 to 850 µm, preferably from 150 to 600 µm.

Preferably, the absorbent pad comprises at least one superabsorbent layer 103b and at least one liquid distribution layer.

As illustrated in figure 3, the absorbent pad 103 may comprise a first liquid distribution layer 103a, a superabsorbent layer 103b and a second liquid distribution layer 103c, wherein the superabsorbent layer 103b is arranged between the first 103a and the second 103c liquid distribution layers.

Preferably, the first 103a or the second 103b liquid distribution layer is a nonwoven layer.

The absorbent pad 103 is configured to absorb wound exudate and to distribute such wound exudate in an efficient manner. In embodiments where the NPWT system comprises a canister, the absorbent pad 103 may function as a temporary reservoir to retain and distribute exudate, while also securing a controlled transport of liquid transport towards the canister via the tubing.

Exudate entering the liquid distribution layer 103a from the wound site is evenly distributed before reaching the superabsorbent layer 103b, thereby creating a larger surface area towards the superabsorbent layer 103b.

In exemplary embodiments, the first liquid distribution layer 103a is arranged below the superabsorbent layer 103b and has a greater liquid spreading capacity than the second liquid distribution layer 103c. An absorbent pad with a liquid spreading gradient is thus achieved, which impacts the ability of the absorbent pad 103 to retain, and remove, respectively, liquid from and within the dressing.

For example, the first liquid distribution layer 103a may comprise a nonwoven. The nonwoven may have a grammage in the range of from 20 to 50 gsm, e.g. from 30 to 40 gsm.

The second liquid distribution layer 103c may be a tissue or a nonwoven layer. Typically, the spreading capability of the second, upper liquid distribution layer 103c is lower than the spreading capability of the lower, first liquid distribution layer 103a.

The layer 103c also serves to prevent leakage of SAP particles from the superabsorbent layer 103b. The SAP particles of the superabsorbent layer 103b chemically bind exudate entering the superabsorbent layer 103b, and thereby forms an aqueous gel. The layer 103c prevents gelling particles from moving towards the backing layer 101 and towards the coupling member 108 comprising the tubing 110. Undesirable blockage of gel particles within the tubing 110 is thereby prevented. The liquid distribution layer 103c creates a larger indirect surface of distributed liquid towards the backing layer 101 of the dressing 100. The layer 103c may also serve as a "support layer" and act as a carrier during the manufacturing process.

The various layers of the absorbent pad create a complex liquid absorption and retention structure resulting in an improved liquid handling and distribution. In embodiments where the NPWT system comprises a remote fluid collection means, a controlled distribution of exudate being retained, and removed from the dressing, respectively, is accomplished.

The absorbent pad 103 may be embossed. In other words, the surface(s) of the absorbent pad 103 is structured and may comprise a plurality of indentations and elevations (not shown). This is beneficial since an absorbent pad 103 comprising a plurality of layers may become stiff and thick as the basis weight increases. The embossing allows the absorbent pad to retain its shape, while being pliable. An embossed absorbent pad 103 also improves the liquid spreading or liquid distribution capacity.

The superabsorbent layer 103b may be an airlaid superabsorbent layer. In embodiments, the airlaid superabsorbent layer 103b comprises superabsorbent particles, cellulosic fibers and bicomponent fibers.

For example, the airlaid superabsorbent layer may comprise:
- 30-50 %, preferably 35-50 % by weight of superabsorbent particles
- 30-50 %, preferably 40-50 % by weight of cellulosic fibers
- 3-10%, preferably 5-8 % by weight of bicomponent fibers
- 3-8% by weight of polyethylene.

Such a superabsorbent layer allows for improved liquid handling properties and a proper distribution of liquid. Furthermore, it prevents gel blocking and prevents the absorbent pad from collapsing when a large amount of fluid is handled.

The bicomponent fibers act as a bonding agent, providing integrity to the SAP layer, especially in the wet state. The biocomponent fibers may be made of polyethylene and polyethylene terephthalate (PE/PET).

In exemplary embodiments, the absorbent pad 103 may comprise additional pad-forming layers (not shown).

To yield extra protection and prevent the backing layer from being punctured by the pile filaments of the spacer fabric layer, both the liquid spreading layer 102 and the nonwoven layer of the absorbent pad 103 may be arranged to overlap the peripheral edges of the top layer 104a of the spacer fabric layer 104.

Accordingly, at least two nonwoven layers are arranged to overlap the peripheral edges of the top layer of the spacer fabric.

As illustrated in figures 2 and 3a-c, the liquid spreading layer 102 and each pad-forming layer of the absorbent pad 103 are arranged to overlap the peripheral edges of the top layer 104a of the spacer fabric layer 104.

This arrangement enhances the protection of the backing layer.

The combined thickness of the liquid spreading layer 102 and the absorbent pad 103 is preferably larger than the thickness, t1, of the spacer fabric layer 104c.

Accordingly, the pile filaments are prevented from extending through the thicker overlapping absorbent pad and the liquid spreading layer.

The thickness of the absorbent pad may be from 2 to 10 mm, e.g. from 3 to 6 mm. The thickness is measured in dry conditions.

The thickness of the liquid spreading layer may be from 0.1 to 2 mm, e.g. from 0.2 to 1 mm. The thickness is measured in dry conditions.

As best illustrated in figure 1, the dressing may comprise a tubing 110, wherein a first distal end of the tubing 110 is connected to the coupling member 108 and a second distal end is configured to be connected to a negative pressure source, wherein the tubing 110 comprises a fluid conduit 110a configured to remove fluid from the dressing and an air conduit 110b configured to supply air to the fluid conduit 110a and/or the NPWT dressing 100.

The NPWT dressing 100 of the present disclosure is preferably adapted for use in an NPWT system comprising a remote fluid collection means.

As used herein, the term "remote fluid collection means" means that the fluid collection means is arranged at a distance from the dressing, e.g. between the dressing and the negative pressure source or is connected to the negative pressure source. The negative pressure source and the remote fluid collection means may be arranged in the same NPWT device. Typically, the remote fluid collection means is a canister.

The tubing 110 is configured to transmit negative pressure to the dressing and to the wound site.

The tubing 110 and/or the coupling member 108 may be of any suitable flexible tubing fabricated from elastomeric and/or polymeric materials. The tubing is attached to the coupling member 108. The tubing 110 may be firmly attached to the coupling member 108 or detachably attached to the coupling member 108.

The coupling member 108 typically comprises an attachment portion configured to be attached to the backing layer of the dressing. The coupling member may be adhesively attached to the backing layer. The coupling member may also comprise a fluid inlet and a fluid outlet configured to be connected to the tubing 110; i.e. to the air conduit 110b, and to the fluid conduit 110a, respectively.

The coupling member may have the construction as defined in EP application No. 13152841.6.

A first distal end of the tubing 110 may be connected to a first connector portion 111a. The first connector portion 111a may be configured to be connected to a second connector portion (not shown) associated with the remote fluid collection means; i.e. the canister and, in embodiments, to the negative pressure source.

In the various embodiments described hereinbefore, the backing layer typically comprises or consists of a polymeric film. The polymeric film may comprise polyurethane, polyamide and/or polyethylene. The backing layer may also be a laminate of polyester based nonwoven materials and at least one polymeric film.

Preferably, the backing layer comprises polyurethane. The backing layer may be a polyurethane film.

The thickness of the backing layer may be in the range of from 10 to 40 µm, preferably from 15 to 30 µm.

The backing layer is the outermost layer of the dressing and is configured to face away from the skin of a wearer. The backing layer may also be referred to as the top layer of the dressing.

The backing layer may have a tensile strength in the machine direction (MD and/or cross-machine direction (CD) of from 30 to 70 MPa, preferably from 35 to 55 MPa, as measured by ISO 527-3/2/200. The tensile strength is measured with 15 mm wide strips.

Preferably, the backing layer 101 has sufficient "strength" to withstand the forces inflicted on the backing layer during movement of the patient, yet allowing for pliability and a sufficient degree of stretchability.

A tensile strength in the above-mentioned range may prevent tearing or rupture of the backing layer. However, the backing layer must still be sufficiently pliable to allow the dressing to adapt to the movement of a user or to the bending of a joint, such as a knee.

A thin layer of adhesive, such as a polyacrylate adhesive, may be applied to the backing layer to attach the backing layer to the adhesive skin contact layer and/or to the liquid spreading layer underlying the backing layer.

In the various embodiments described hereinbefore, the adhesive skin contact layer preferably comprises a silicone-based adhesive, e.g. a silicone gel.

An adhesive skin contact layer comprising a silicone gel is skin-friendly and easy to remove without causing trauma. It is sufficiently adherent to skin such that the dressing stays in place, yet is configured to maintain its adherence with repeated removal and re-application.

As illustrated in e.g. figure 3a, the adhesive skin contact layer 105 may comprise two layers. For example, the adhesive skin contact layer 105 may comprise a polymeric film 105a and a silicone gel layer 105b; the silicone gel layer 105b being arranged to contact the skin of a wearer.

The polymeric film 105a is preferably a breathable film. The polymeric film may comprise e.g. polyethylene, polyamide, polyester or polyurethane. Preferably, the polymeric film comprises polyurethane.

The thickness of the polymeric film may be from 15 to 100 µm, e.g. from 20 to 80 µm, preferably from 20 to 60 µm.

Examples of suitable silicone gels for use in the adhesive skin contact layer 105 include the two component RTV systems, such as Q72218 (Dow Corning), and SilGel 612 (Wacker Chemie AG) mentioned herein, as well as NuSil silicone elastomers. In embodiments of the invention the adhesive may comprise a silicone gel having a softness (penetration) of from 8 to 22 mm, e.g. from 12 to 17 mm, as measured by a method based on ASTM D 937 and DIN 51580, the method being described in European Patent Application No 14194054.4.

The thickness of the adhesive skin contact layer is typically at least 20 µm. Typically, the thickness of the adhesive skin contact layer is from 100 to 200 µm.

In preferred embodiments, and as best illustrated in figure 3, the adhesive skin contact layer 105 comprises a plurality of perforations 119 in the area underlying the absorbent pad 103, but is void of perforations in the area forming the border portion 106.

The lack of perforations in the border portion 106 of the dressing is beneficial to improve the adhesion at the borders of the dressing and thereby improve the stay-on ability of the dressing.

In embodiments where the adhesive skin contact layer 105 comprises a polymeric film 105a and a silicone gel layer 105b, the perforations 119 extend through the polymeric film 105a and the silicone gel layer 105b.

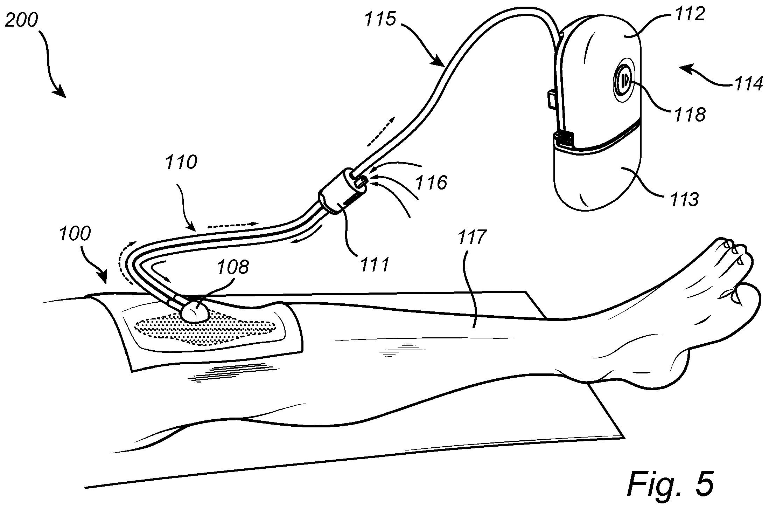

Figure 5 conceptually illustrates a negative pressure wound therapy (NPWT) system according to the present disclosure.

The negative pressure wound therapy (NPWT) system 200 comprises an NPWT dressing 100 as described hereinbefore. The dressing 100 is applied to the knee of a patient 117.

The NPWT system 200 comprises
- a negative pressure wound therapy (NPWT) dressing 100 as described hereinbefore,
- a negative pressure source
- a remote fluid collection means 113 fluidly connected to the negative pressure source and to the dressing 100.

The negative pressure source may be a negative pressure pump adapted for establishing a negative pressure when the negative pressure pump is in an active state. The negative pressure pump may be any type of pump that is biocompatible and maintains or draws adequate and therapeutic vacuum levels. Preferably, the negative pressure level to be achieved is in a range between about -20 mmHg and about -300 mmHg. In embodiments of the present disclosure, a negative pressure range between about -80 mmHg and about -180, preferably between about -100 and - 150 mmHg, more preferably between -110 and -140 mmHg is used.

The negative pressure pump may be a pump of the diaphragmatic or peristaltic type.

As used herein, the term "fluidly connected" should be interpreted broadly and may comprise e.g. any form of tubing, conduits, or channels providing a fluid connection/communication between the remote fluid collection means 113 and the negative pressure source and the dressing 100.

The remote fluid collection means 113 may be any kind of fluid container, such as a canister. Alternatively, it may be an absorbent material present within the tubing of the NPWT dressing or NPWT system or a dressing or absorbent pad arranged between the dressing of the present disclosure and the canister. Typically, the remote fluid collection means 113 is a canister.

In figure 5, the negative pressure source and the canister 113 are arranged in the same device 114. The negative pressure source is comprised within a housing 112 of the device 114. The device 114 is typically a portable device.

The canister 113 is preferably detachably connected to the housing 112.

In other words, the canister 113 is releasably connected to the housing 112. The detachable connection may be by conventional means including a friction fit, bayonet coupling, snap fit, barbed connector, or the like. The detachable configuration allows the user or caregiver to remove the canister 113 and empty the collected liquid, and subsequently re-attach the canister 113 to the housing 112 again.

The canister 113 may be formed from e.g. molded plastic or the like. The canister 113 is preferably at least partly transparent/translucent to permit viewing into the interior of the canister 113 to assist the user in determining the remaining capacity of the canister 113.

For example, an inner volume of the canister 113 may be between 30 - 300 ml, e.g between 40 and 150 ml. The inner volume of the canister 103 may vary depending on the type of wound. In embodiments, the canister 113 comprises a liquid absorbent material. In a possible embodiment at least 75% of the inner volume of the canister 103 is occupied with a liquid absorbent material.

The NPWT device 114 may be connected to the NPWT dressing 100 by means of the tubing 110.

In the embodiment illustrated in figure 5, the NPWT system comprises a connector unit 111 at a position between the dressing 100 and the NPWT device 114. The connector unit 111 may comprise the first connector portion (denoted 111a in figure 1) and a second connector portion (not shown) associated with tubing of the canister and/or the negative pressure source. The first and second connector portions are preferably detachably connected such that the dressing can be easily disconnected from the NPWT device 114. This is beneficial in portable NPWT systems as the user may decide to disconnect the dressing from the device 114 when he/she is going to take a shower or for some other reason.

In figure 5, the tubing 110 is a double conduit, whereas the tubing 115 between the NPWT device 114 and the connector unit 111 is a single conduit. The NPWT system is by no means limited to such a construction, but may comprise a single conduit or a double conduit between the NPWT device 114 and the dressing 100. The NPWT system is also not limited to the use of a connector unit 111. The tubing 110 may, in embodiments be configured to extend all the way to the NPWT device 114.

The NPWT system 200 preferably comprises means to supply air to the dressing at a rate of from 2 to 7 ml/min during operation.

Preferably, the means to supply air to the dressing is configured to supply air at a rate of from 2-7 ml, preferably of from 3-5 ml at a negative pressure of from -80 to -180 mmHg, preferably of from -100 to -150 mmHg, more preferably of from -110 to -140 mmHg.

In the NPWT system 200 illustrated in figure 5, ambient air is introduced into the system by means of the connector unit 111 (illustrated by the arrows 116). For example, the first and/or the second connector portion may comprise an air filter (not shown) configured to control the supply of air into the dressing 100 and/or into the tubing 110. The first and/or the second connector portion may e.g. comprise an air inlet port, wherein the air filter is arranged.

The air filter may comprise a hydrophobic and porous material, wherein the size of the pores is within the range of from 2 to 20 µm, preferably in the range of from 5 to 12 µm. The pore size of the filter is measured in a non-compressed state.

The air filter preferably comprises polyethylene, preferably sintered polyethylene.

The air filter secures that the supply of air is in the range of from 2-7 ml/min during operation, e.g. at a negative pressure of - 80 mmHg to -150 mm Hg, e.g. from -100 mmHg to -130 mmHg.

It should be noted that air may be introduced into the system in alternative ways, and an air filter may be provided at alternative positions in the system. The regulation of air supply may, in embodiments, be controlled by the NPWT device 114.

During use, the dressing 100 is arranged at a wound site of the user/patient 117, forming a sealed space. The tubings (110 and 115) are provided to fluidly connect the dressing 100 to the NPWT device 114, e.g. to an inlet port of the NPWT device 114. The NPWT device 114 is then activated, e.g. by the caregiver or user, by pressing the start/pause button 118. The negative pressure pump is thereby activated. When activated, the negative pressure pump will start to evacuate air through the canister 113, the tubing (110 and 115) and the sealed space formed by the dressing 100. Accordingly, the negative pressure will be created within the sealed space. If wound liquid is present at the wound site, this liquid may at least partly be "drawn" from the wound site, through the tubing (110 and 115), and into the canister 113. The amount of liquid; i.e. exudate that is drawn from the wound and collected in the canister 113 will depend on the type of wound that is being treated as well as the type of wound dressing used.

Within the context of the present disclosure, a substantially equal balance between liquid distribution is desired. A suitable filter member (not shown) may be arranged between the canister 113 and the negative pressure pump to ensure that no liquid is allowed to pass to the negative pressure pump from the canister 113.

According to another aspect, there is provided a method for manufacturing a negative pressure wound therapy (NPWT) dressing, wherein the method comprises:
a) providing a backing layer 101,
b) arranging a liquid spreading layer 102 in contact with the backing layer 101,
c) arranging an absorbent pad 103 comprising one or a plurality of pad-forming layers 103a-c in contact with the liquid spreading layer 102,
d) arranging a spacer fabric layer 104 in contact with the absorbent pad 103, wherein the spacer fabric layer 104 comprises a top layer 104a, a bottom layer 104b and an interconnecting layer 104c arranged between the top layer 104a and the bottom layer 104b; the interconnecting layer 104c having a height, h1, and comprising a plurality of pile filaments 107 extending between the top layer 104a and the bottom layer 104b, wherein the top layer 104a of the spacer fabric layer 104 is defined by peripheral edges,
e) arranging an adhesive skin contact layer 105 in contact with the spacer fabric layer 104, wherein the adhesive skin contact layer 105 and the backing layer 101 are arranged to extend beyond the contour of the liquid spreading layer 102, the absorbent pad 103 and the spacer fabric layer 104 to form a border portion 106, and
wherein at least one of the liquid spreading layer and the pad-forming layer(s) of the absorbent pad 103 is arranged to overlap the peripheral edges of the top layer 104a of the spacer fabric layer 104.

The method may be automated, semi-automated or manual.

The various layers may be attached by means known in the art. For example, the layers may be adhesively attached. In exemplary embodiments, a hot-melt adhesive, such as polyacrylate is used to attach the dressing layers with one another. In the border portion 106, the backing layer 101 and the adhesive skin contact layer 105 may be adhesively attached.

The liquid spreading layer 102, the absorbent pad 103 and the spacer fabric layer 104 are typically cut into shape prior to the process of manufacturing the NPWT dressing of the present disclosure.

To improve the conformability of the backing layer to the pad edges, the backing layer 101 may be pre-formed to a shape complementary to the shape of at least the absorbent pad 103 prior to or simultaneously with arranging the liquid spreading layer 102 in contact with the backing layer 101.

In exemplary embodiments, the backing layer 101 is pre-formed by
a) contacting the backing layer 101 with a molding tool having a shape complementary to at least the absorbent pad 103, and
b) applying vacuum to the backing layer 101.

The molding tool may have a depth corresponding to the combined thickness of the backing layer, the liquid spreading layer, the absorbent pad, and the spacer fabric layer.

The molding tool may have a width and length corresponding to the width and length of the liquid spreading layer and the absorbent pad.

The surface of the molding tool corresponds to the shape of the central portion of the backing layer into which the liquid spreading layer, the absorbent pad, and the spacer fabric layer are to be arranged.

Preferably, the molding tool comprises a foam. This is to prevent the backing layer from rupture during assembly of the dressing.

The step of applying vacuum to the backing layer 101 draws the backing layer 101 into the molding tool such that the central portion of the backing layer 101 adopts the shape of the molding tool.

It is also conceivable to apply heat during the step b) to improve the process of pre-forming the backing layer.

The steps b)-e) of arranging the liquid spreading layer 102, the absorbent pad 103, and the spacer fabric layer 104 may be performed simultaneously with contacting the backing layer 101 with a molding tool having a shape complementary to the shape of at least the absorbent pad 103, and, preferably, while applying vacuum to the backing layer 101.

The application of vacuum allows the backing layer to be stretched, and to adopt a pre-formed shape that matches that of the absorbent pad, and preferably also the liquid spreading layer.

According to yet another aspect, there is provided a kit comprising the negative pressure wound therapy (NPWT) dressing as described hereinbefore, and at least one additional component selected from a negative pressure source, a canister, a battery and/or adhesive strip(s).

The NPWT dressing preferably comprises a pre-attached tubing 110. This allows for a quick assembly of the components of the kit.

The kit may further comprise a negative pressure device 114. The negative pressure device 114 may comprise a negative pressure source and a canister.

The kit may comprise additional components such as additional batteries for powering the NPWT device 114 and adhesive strips for improving the adhesion between the border portion of the dressing to the skin of a wearer.

The kit may be adapted for home care, but may also be advantageously used in a hospital or a care facility setting. The NPWT device is adapted to be carried by the user, e.g. in a pocket, belt, strap or similar. The NPWT dressing and the other components of the kit can easily be assembled by a user.

The NPWT device 114 used in the kit (and in the NPWT system) of the present disclosure comprises the features and components necessary to control the operation of the device. For example, the NPWT device may comprise a control unit electrically connected to a battery. Such a control unit may comprise a microprocessor, microcontroller, programmable digital signal processor or another programmable device. In addition, the NPWT device 114 may comprise at least one pressure sensor arranged in fluid connection with the negative pressure pump.

### Examples

### Example 1: Evaluation of backing layer rupture in comparative wear tests

Wear tests were carried out utilizing a dressing according to the present disclosure (having the general construction as illustrated in figure 3a), referred to hereinafter as Dressing A, and a comparative dressing (having the general construction as illustrated in figure 4a), referred to hereinafter as Dressing B.

The dressings were similar in construction and differed only with respect to the arrangement of the spacer fabric layer.

Dressing A and B comprised, from bottom-to-top, an adhesive skin contact layer comprising a polyurethane film and a silicone gel layer, a spacer fabric layer, an absorbent pad (comprising a nonwoven liquid spreading layer, a superabsorbent layer and a tissue layer), a nonwoven liquid spreading layer and a backing layer, respectively. Both dressings comprised a pre-attached tubing comprising an air conduit and a fluid conduit.

In Dressing A, the absorbent pad and the liquid spreading layer were arranged to overlap the spacer fabric layer with 3 mm. The distance was measured from the peripheral edges of the top layer of the spacer fabric layer.

In Dressing B, the absorbent pad, the liquid spreading layer and the spacer fabric layer were co-extensive in length and width.

The thickness of the spacer fabric layer (in Dressings A and B) was 2.4 mm.

The dressings were attached to the front knees of a number of test subjects with the leg bent at 120 degrees (the dressing tubing pointing upwards). The tubing was connected to a portable negative pressure device by means of a connector (denoted 111 in figure 5). The pump used was of diaphragmic type. A canister configured to store 50 ml of liquid (as disclosed in figure 5) was connected to the pump. The connector portion attached to the distal end of the dressing tubing comprises an air filter and ambient air was introduced into the connector and into the system such that the supply of air to the dressing (by means of the conduit) was within the range of 2-7 ml/min during the operation.

The pump was activated, and a negative pressure of 125 mmHg was applied to the dressings.

Tests were performed on 14 subjects for dressing A and 10 subject for dressing B.

The dressings were worn on the test subjects' knees for 72 hours and subjected to movements and stress. At the end of the test period, the dressings were evaluated regarding micro leakages, i.e. micro holes created in the backing layer during the wear time.

In order to identify the formation of micro holes in the backing layer, each dressing sample was wetted with colored liquid from the outside of the backing layer. By means of the applied negative pressure in the wound pad the liquid easily enters a hole formed in the backing layer and stains/colors the wound pad, which enables visual identification of micro leakages.

As illustrated in table 1 hereinbelow, a significant improvement in preventing backing layer rupture was observed with a dressing according to the present disclosure (Dressing A).

**Table 1: Micro hole formation in the backing layer after 72 h wear time**

| | Dressing A | Dressing B |
|---|---|---|
| Number of dressings with holes formed in backing layer after 72 h wear time | 3 of 14 (21%) | 10 of 10 (100%) |

### Example 2: Evaluation of backing layer rupture in simulated wear tests

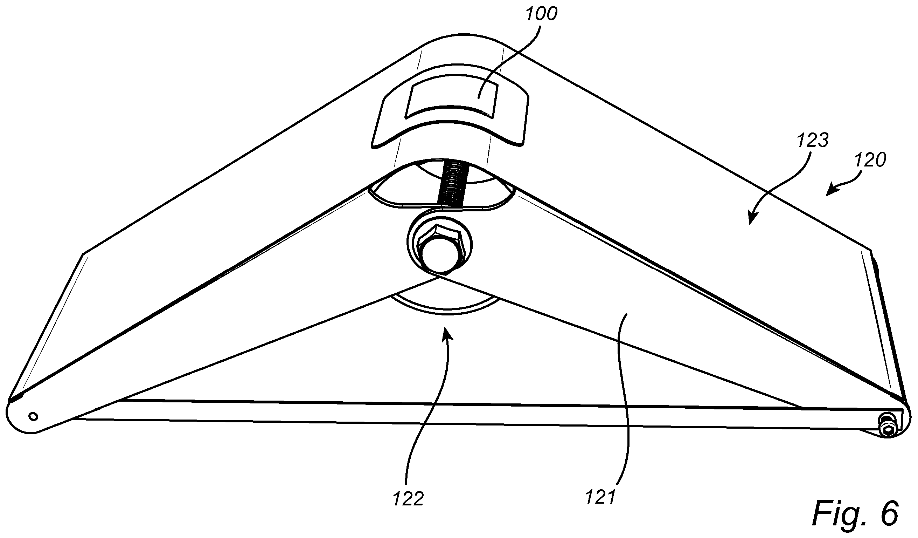

A simulated wear test was also carried out, wherein the dressings (Dressings A and B) were stressed and flexed repeatedly by means of the test equipment illustrated in figure 6.

The test equipment 120 comprised a plastic plate 121 with a cylinder hinge 122 of a size to mimic an articulating joint, i.e. a knee. A polyurethane plastic film 123 was attached to the upper surface of the plate 121 to mimic the skin and to enable an airtight seal. The hinge was attached to a tensile tester to allow the hinge to cycle in a consistent and predefined pace.

The dressing 100 was applied at an angle of the hinge at 120 degrees.

The tubing was connected to a negative pressure device in the same manner as described in Example 1.

The supply of air to the dressing (by means of the conduit) was within the range of 2-7 ml/min during the operation. The pump was activated, and a negative pressure of 125 mmHg was applied to the dressings.

The hinge was cycled 250 times or until leakage occured, at a pace of 2000 mm/min, and varying the hinge from a first position at an angle of 15 degrees to a second position at an angle of 180 degrees. The hinge stayed in each position for one second. Tests were performed with 5 dressings in each dressing category (Dressing A, and B, respectively)

At predefined intervals between the cycles (every 10^{th} cycle up to 100 then 50 at a time), the dressings were investigated regarding micro leakages, i.e. small holes created in the backing layer during the wear time. In order to identify backing layer rupture, each dressing sample was wetted with colored liquid from the outside of the backing layer as described hereinbefore with respect to Example 1.

As illustrated in table 2 hereinbelow, a significant improvement in preventing rupture of the backing layer was observed with a dressing according to the present disclosure (Dressing A).

**Table 2: Micro hole formation in the backing layer in simulated wear test**

| | Dressing A | Dressing B |
|---|---|---|
| Number of dressings with holes formed in backing layer after 250 cycles in simulated wear test | 0 of 5 (0%) | 4 of 5 (80%) |

### Example 3: Evaluation of negative pressure decay

Negative pressure decay was measured after repeatedly turning the negative pressure source on and off with respect to the dressing A and dressing B. This was to stress the backing layer and force the wound pad to repeated contact with the backing layer.

The dressing was applied to a Plexiglas plate. The tubing was connected to a mobile negative pressure device in the same manner described in Example 1.

A pressure sensor source was connected to the dressing in order to measure the negative pressure in the dressing and system.

The pump was activated, and a negative pressure of 125 mmHg was applied to the dressings. The negative pressure source was turned off and after 10 minutes the dressing was tested for micro holes with colored liquid from the outside of the backing layer, in the same manner as described with respect to Example 1.

This procedure was repeated 20 cycles with 125 mmHg and then with three additional cycles with 180 mmHg.

Five dressings in each dressing category (A and B) were evaluated.

Similar results with respect to micro hole formation in the backing layer as in Example 2, was obtained (see table 3 hereinbelow).

For the dressings where the backing layer was punctured (Dressing B), there was a significant decay in negative pressure (see table 3).

Hence, with a dressing according to the present disclosure (Dressing A), the backing layer is prevented from becoming ruptured after stressing the dressing with repeated cycling of adding and removing negative pressure. Furthermore, no significant decay in negative pressure is observed.

**Table 3: Negative pressure decay and micro hole formation in the backing layer**

| | Dressing A | Dressing B |
|---|---|---|
| Average pressure decay | 0.2 mm Hg | 2.5 mm Hg |
| Number of dressings with micro holes in backing layer after 20 cycles with 125 mmHg | 0 of 5 (0%) | 4 of 5 (80%) |
| Number of dressings with micro holes in backing layer after 20 cycles with 125 mmHg + 3 cycles with 180 mmHg | 0 of 5 (0%) | 4 of 5 (80%) |

### Example 4: Negative pressure distribution

System tests were carried out to ensure that the negative pressure is evenly distributed to the wound bed even though the area of the spacer fabric layer is decreased. The spacer fabric layer acts as a transmission layer for negative pressure in the dressings. Tests were carried out to evaluate whether the negative pressure distribution would be compromised with a spacer fabric layer of a smaller size (Dressing A).

Negative pressure was applied to the dressings located on a wound model, i.e. a Plexiglas plate. The Plexiglas plate was equipped with sensors for measuring the negative pressure level, and inlet holes to provide the dressings with test liquid in order to mimic normal use. The liquid was supplied from underneath the dressing using a peristaltic pump (providing liquid with a defined flow rate). The method evaluates the negative pressure distribution from the pressure source to the different parts of a dressing.

Tests were carried out during 96 hours with 5 dressings in each of the dressing categories (dressing A, and B, respectively).

The results, as illustrated in table 5 hereinbelow, demonstrate that both dressings A and B manage to transmit the negative pressure efficiently. There is no significant difference between the measured level of negative pressure between dressing A and B.

**Table 5: Transmission of negative pressure**

| | Dressing A | Dressing B |
|---|---|---|
| Negative pressure at the bottom of the dressing during 96 h | 122,5 mmHg | 123,9 mmHg |

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A negative pressure wound therapy (NPWT) dressing (100) configured to be connected to a negative pressure source; said NPWT dressing comprising, from top-to-bottom, a backing layer (101), a liquid spreading layer (102), an absorbent pad (103) comprising one or a plurality of pad-forming layers (103a-c), a spacer fabric layer (104), and an adhesive skin contact layer (105); said backing layer (101) and said adhesive skin contact layer (105) being arranged to extend beyond the contour of said liquid spreading layer (102), said absorbent pad (103) and said spacer fabric layer (104) to form a border portion (106), wherein said spacer fabric layer (104) comprises a top layer (104a), a bottom layer (104b) and an interconnecting layer (104c) arranged between said top layer (104a) and said bottom layer (104b); said interconnecting layer (104c) comprising a plurality of pile filaments (107) extending between said top layer (104a) and said bottom layer (104b), wherein said top layer (104a) of said spacer fabric layer (104) is defined by peripheral edges, and wherein at least one of said liquid spreading layer (102) and said pad-forming layer(s) (103a-c) of said absorbent pad (103) is arranged to overlap said peripheral edges of said top layer (104a) of said spacer fabric layer (104).

2. The NPWT dressing (100) according to claim 1, wherein said backing layer (101) has a central portion and an edge portion surrounding said central portion, wherein said central portion has a pre-formed shape complementary to the shape of at least said absorbent pad (103), and wherein said edge portion forms part of said border portion (106).

3. The NPWT dressing (100) according to claim 1 or claim 2, wherein said spacer fabric layer (104) has a thickness, t1, and wherein at least one of said liquid spreading layer (102) and said pad-forming layer(s) (103a-c) of said absorbent pad (103) is arranged to overlap said peripheral edges of said top layer (104a) by a distance, d1, wherein said distance d1 corresponds to 0.7 to 4.0t1, preferably 1.0 to 2.0t1.

4. The NPWT dressing (100) according to claim 1, further comprising a coupling member (108) configured to connect said dressing to a negative pressure source, wherein said backing layer (101) and at least a portion of said absorbent pad (103) comprises an opening (109) underneath said coupling member (108); said liquid spreading layer (102) being void of an opening.

5. The NPWT dressing (100) according to claim 1, wherein said liquid spreading layer (102) is a continuous layer extending across the entire surface area of said absorbent pad (103).

6. The NPWT dressing (100) according to any one of the preceding claims, wherein said liquid spreading layer (102) is a nonwoven layer.

7. The NPWT dressing (100) according to any one of the preceding claims, wherein said absorbent pad (103) comprises a first liquid distribution layer (103a), a superabsorbent layer (103b) and a second liquid distribution layer (103c), wherein said superabsorbent layer (103b) is arranged between said first (103a) and said second (103c) liquid distribution layers, and wherein said first (103a) or said second (103b) liquid distribution layer is a nonwoven layer.

8. The NPWT dressing (100) according to any one of the preceding claims, wherein both said liquid spreading layer (102) and said nonwoven layer of said absorbent pad (103) are arranged to overlap said peripheral edges of said top layer (104a) of said spacer fabric layer (104).

9. The NPWT dressing (100) according to any one of the preceding claims, wherein said liquid spreading layer (102) and each pad-forming layer (103a-c) of said absorbent pad (103) are arranged to overlap said peripheral edges of said top layer (104a) of said spacer fabric layer (104).

10. The NPWT dressing (100) according to claim 9, wherein the combined thickness of said liquid spreading layer (102) and said absorbent pad (103) is larger than the thickness, t1, of said spacer fabric layer (104c).

11. The NPWT dressing (100) according to any one of the preceding claims, wherein said dressing comprises a tubing (110), wherein a first distal end of said tubing (110) is connected to said coupling member (108) and a second distal end of said tubing (110) is configured to be connected to a negative pressure source, wherein said tubing (110) comprises a fluid conduit (110a) configured to remove fluid from said dressing and an air conduit (110b) configured to supply air to said fluid conduit (110a) and/or said NPWT dressing (100).

12. A negative pressure wound therapy (NPWT) system (200) comprising:
- a negative pressure wound therapy (NPWT) dressing (100) according to any one of claims 1-11,
- a negative pressure source,
- a remote fluid collection means (113) fluidly connected to said negative pressure source and to said dressing (100).

13. The NPWT system (200) according to claim 12, wherein said remote fluid collection means (113) is a canister and wherein said canister and said negative pressure source are arranged within the same device (114).

14. The NPWT system (200) according to claim 13, wherein said system (200) comprises means to supply air to said dressing (100) at a rate of from 2 to 7 ml/min during operation.

15. A method for manufacturing a negative pressure wound therapy (NPWT) dressing (100), wherein said method comprises:
a) providing a backing layer (101),
b) arranging a liquid spreading layer (102) in contact with said backing layer (101),
c) arranging an absorbent pad (103) comprising one or a plurality of pad-forming layers (103a-c) in contact with said liquid spreading layer (102),
d) arranging a spacer fabric layer (104) in contact with said absorbent pad (103), wherein said spacer fabric layer (104) comprises a top layer (104a), a bottom layer (104b) and an interconnecting layer (104c) arranged between said top layer (104a) and said bottom layer (104b); said interconnecting layer (104c) comprising a plurality of pile filaments (107) extending between said top layer (104a) and said bottom layer (104b), wherein said top layer (104a) of said spacer fabric layer (104) is defined by peripheral edges,
e) arranging an adhesive skin contact layer (105) in contact with said spacer fabric layer (104), wherein said adhesive skin contact layer (105) and said backing layer (101) are arranged to extend beyond the contour of said liquid spreading layer (102), said absorbent pad (103) and said spacer fabric layer (104) to form a border portion (106), and
wherein at least one of said liquid spreading layer and said pad-forming layer(s) of said absorbent pad (103) is arranged to overlap said peripheral edges of said top layer (104a) of said spacer fabric layer (104).

16. The method according to claim 15, wherein said backing layer (101) is pre-formed to a shape complementary to the shape of at least said absorbent pad (103) prior to or simultaneously with the step b) of arranging said liquid spreading layer in contact with said backing layer (101).

17. The method according to claim 16, wherein said backing layer (101) is pre-formed by
a) contacting said backing layer (101) with a molding tool having a shape complementary to the shape of at least said absorbent pad (103), and
b) applying vacuum to said backing layer (101).

18. The method according to claim 17 when dependent on claim 15, wherein said steps b)-d) of arranging said liquid spreading layer (102), said absorbent pad (103), and said spacer fabric layer (104 are performed simultaneously with contacting said backing layer (101) with a molding tool having a shape complementary to the shape of said liquid spreading layer (102) and said absorbent pad (103), and preferably while simultaneously applying vacuum to said backing layer (101).

## Patentansprüche

1. Verband (100) für die Unterdruckwundtherapie (UWT), der so ausgelegt ist, dass er mit einer Unterdruckquelle verbunden wird; wobei der UWT-Verband von oben nach unten Folgendes umfasst: eine Trägerschicht (101), eine Flüssigkeitsausbreitungsschicht (102), eine absorbierende Auflage (103), die eine oder eine Vielzahl von auflagenbildende(n) Schicht(en) (103a-c) bildet, eine Abstandsstoffschicht (104) sowie eine haftende Hautkontaktschicht (105); wobei die Trägerschicht (101) und die haftende Hautkontaktschicht (105) so angeordnet sind, dass sie sich über den Umriss der Flüssigkeitsausbreitungsschicht (102), der absorbierenden Auflage (103) und der Abstandsstoffschicht (104) hinaus erstrecken und somit einen Grenzabschnitt (106) bilden, wobei die Abstandsstoffschicht (104) eine obere Schicht (104a), eine untere Schicht (104b) und eine Verbindungsschicht (104c) umfasst, die zwischen der oberen Schicht (104a) und der unteren Schicht (104b) angeordnet ist; wobei die Verbindungsschicht (104c) eine Vielzahl von Stapelfilamenten (107) umfasst, die zwischen der oberen Schicht (104a) und der unteren Schicht (104b) verlaufen, wobei die obere Schicht (104a) der Abstandsstoffschicht (104) durch umlaufende Ränder definiert ist und wobei die Flüssigkeitsausbreitungsschicht (102) und/oder die auflagenbildende(n) Schicht(en) (103a-c) der absorbierenden Auflage (103) so angeordnet ist bzw. sind, dass sie die umlaufenden Ränder der oberen Schicht (104a) der Abstandsstoffschicht (104) überdeckt bzw. überdecken.

2. UWT-Verband (100) nach Anspruch 1, wobei die Trägerschicht (101) einen mittleren Abschnitt und einen Randabschnitt aufweist, der den mittleren Abschnitt umgibt, wobei der mittlere Abschnitt eine vorgeformte Form aufweist, die ergänzend zu der Form von zumindest der absorbierenden Auflage (103) ist, und wobei der Randabschnitt einen Teil des Grenzabschnitts (106) bildet.

3. UWT-Verband (100) nach Anspruch 1 oder Anspruch 2, wobei die Abstandsstoffschicht (104) eine Dicke, t1, aufweist und wobei die Flüssigkeitsausbreitungsschicht (102) und/oder die auflagenbildende(n) Schicht(en) (103a-c) der absorbierenden Auflage (103) so angeordnet ist bzw. sind, dass sie die umlaufenden Ränder der oberen Schicht (104a) um einen Abstand, d1, überdeckt bzw. überdecken, wobei der Abstand d1 0,7 bis 4,0 t1, vorzugsweise 1,0 bis 2,0 t1 entspricht.

4. UWT-Verband (100) nach Anspruch 1, ferner umfassend ein Kopplungselement (108), das so ausgelegt ist, dass es den Verband mit einer Unterdruckquelle verbindet, wobei die Trägerschicht (101) und zumindest ein Abschnitt der absorbierenden Auflage (103) eine Öffnung (109) unter dem Kopplungselement (108) umfasst; wobei die Flüssigkeitsausbreitungsschicht (102) keine Öffnung aufweist.

5. UWT-Verband (100) nach Anspruch 1, wobei die Flüssigkeitsausbreitungsschicht (102) eine durchgängige Schicht ist, die sich über die gesamte Oberfläche der absorbierenden Auflage (103) erstreckt.

6. UWT-Verband (100) nach einem der vorhergehenden Ansprüche, wobei die Flüssigkeitsausbreitungsschicht (102) eine Vliesschicht ist.

7. UWT-Verband (100) nach einem der vorhergehenden Ansprüche, wobei die absorbierende Auflage (103) eine erste Flüssigkeitsverteilungsschicht (103a), eine superabsorbierende Schicht (103b) und eine zweite Flüssigkeitsverteilungsschicht (103c) umfasst, wobei die superabsorbierende Schicht (103b) zwischen der ersten (103a) und der zweiten (103c) Flüssigkeitsverteilungsschicht angeordnet ist, und wobei die erste (103a) oder die zweite (103b) Flüssigkeitsverteilungsschicht eine Vliesschicht ist.

8. UWT-Verband (100) nach einem der vorhergehenden Ansprüche, wobei sowohl die Flüssigkeitsausbreitungsschicht (102) als auch die Vliesschicht der absorbierenden Auflage (103) so angeordnet sind, dass sie die umlaufenden Ränder der oberen Schicht (104a) der Abstandsstoffschicht (104) überdecken.

9. UWT-Verband (100) nach einem der vorhergehenden Ansprüche, wobei die Flüssigkeitsausbreitungsschicht (102) und jede auflagenbildende Schicht (103a-c) der absorbierenden Auflage (103) so angeordnet sind, dass sie die umlaufenden Ränder der oberen Schicht (104a) der Abstandsstoffschicht (104) überdecken.

10. UWT-Verband (100) nach Anspruch 9, wobei die zusammengefasste Dicke der Flüssigkeitsausbreitungsschicht (102) und der absorbierenden Auflage (103) größer ist als die Dicke, t1, der Abstandsstoffschicht (104c).

11. UWT-Verband (100) nach einem der vorhergehenden Ansprüche, wobei der Verband einen Schlauch (110) umfasst, wobei ein erstes distales Ende des Schlauchs (110) mit dem Kopplungselement (108) verbunden ist und ein zweites distales Ende des Schlauchs (110) so ausgelegt ist, dass es mit einer Unterdruckquelle verbunden wird, wobei der Schlauch (110) eine Flüssigkeitsleitung (110a), die so ausgelegt ist, dass sie Flüssigkeit aus dem Verband entfernt, und eine Luftleitung (110b) umfasst, die so ausgelegt ist, dass sie Luft zu der Flüssigkeitsleitung (110a) und/oder dem UWT-Verband (100) leitet.

12. System (200) für die Unterdruckwundtherapie (UWT), umfassend:
- einen Verband (100) für die Unterdruckwundtherapie (UWT) nach einem der Ansprüche 1 bis 11,
- eine Unterdruckquelle,
- ein entfernt befindliches Flüssigkeitsauffangmittel (113), das fluidmäßig mit der Unterdruckquelle und dem Verband (100) verbunden ist.

13. UWT-System (200) nach Anspruch 12, wobei das entfernt befindliche Flüssigkeitsauffangmittel (113) ein Kanister ist und wobei der Kanister und die Unterdruckquelle in derselben Vorrichtung (114) angeordnet sind.

14. UWT-System (200) nach Anspruch 13, wobei das System (200) Mittel zum Leiten von Luft zu dem Verband (100) mit einem Wert von 2 bis 7 ml/min während des Betriebs umfasst.

15. Verfahren zum Herstellen eines Verbands (100) für die Unterdruckwundtherapie (UWT), wobei das Verfahren Folgendes umfasst:
a) Bereitstellen einer Trägerschicht (101),
b) Anordnen einer Flüssigkeitsausbreitungsschicht (102) im Kontakt mit der Trägerschicht (101),
c) Anordnen einer absorbierenden Auflage (103), die eine oder eine Vielzahl von auflagenbildende(n) Schicht(en) (103a-c) umfasst, im Kontakt mit der Flüssigkeitsausbreitungsschicht (102),
d) Anordnen einer Abstandsstoffschicht (104) im Kontakt mit der absorbierenden Auflage (103), wobei die Abstandsstoffschicht (104) eine obere Schicht (104a), eine untere Schicht (104b) und eine Verbindungsschicht (104c) umfasst, die zwischen der oberen Schicht (104a) und der unteren Schicht (104b) angeordnet ist; wobei die Verbindungsschicht (104c) eine Vielzahl von Stapelfilamenten (107) umfasst, die zwischen der oberen Schicht (104a) und der unteren Schicht (104b) verlaufen, wobei die obere Schicht (104a) der Abstandsstoffschicht (104) durch umlaufende Ränder definiert ist,
e) Anordnen einer haftenden Hautkontaktschicht (105) im Kontakt mit der Abstandsstoffschicht (104), wobei die haftende Hautkontaktschicht (105) und die Trägerschicht (101) so angeordnet sind, dass sie sich über den Umriss der Flüssigkeitsausbreitungsschicht (102), der absorbierenden Auflage (103) und der Abstandsstoffschicht (104) hinaus erstrecken und somit einen Grenzabschnitt (106) bilden, und
wobei die Flüssigkeitsausbreitungsschicht und/oder die auflagenbildende(n) Schicht(en) der absorbierenden Auflage (103) so angeordnet ist bzw. sind, dass sie die umlaufenden Ränder der oberen Schicht (104a) der Abstandsstoffschicht (104) überdeckt bzw. überdecken.

16. Verfahren nach Anspruch 15, wobei die Trägerschicht (101) vor dem oder gleichzeitig mit dem Schritt b) zum Anordnen der Flüssigkeitsausbreitungsschicht im Kontakt mit der Trägerschicht (101) in eine Form vorgeformt wird, die ergänzend zu der Form von zumindest der absorbierenden Auflage (103) ist.

17. Verfahren nach Anspruch 16, wobei die Trägerschicht (101) dadurch vorgeformt wird, dass
a) die Trägerschicht (101) mit einem Formwerkzeug in Kontakt gebracht wird, das eine Form aufweist, die ergänzend zu der Form von zumindest der absorbierenden Auflage (103) ist, und
b) Anlegen von Vakuum an die Trägerschicht (101).

18. Verfahren nach Anspruch 17, wenn er von Anspruch 15 abhängt, wobei die Schritte b)-d) zum Anordnen der Flüssigkeitsausbreitungsschicht (102), der absorbierenden Auflage (103) und der Abstandsstoffschicht (104) gleichzeitig damit durchgeführt werden, dass die Trägerschicht (101) mit einem Formwerkzeug in Kontakt gebracht wird, das eine Form aufweist, die ergänzend zu der Form der Flüssigkeitsausbreitungsschicht (102) und der absorbierenden Auflage (103) ist, und vorzugsweise während gleichzeitig Vakuum an die Trägerschicht (101) angelegt wird.

## Revendications

1. Pansement (100) pour thérapie des plaies par pression négative (NPWT) configuré pour être connecté à une source de pression négative ; ledit pansement pour thérapie par pression négative comprenant, de haut en bas, une couche de support (101), une couche d'étalement de liquide (102), un tampon absorbant (103) comprenant une ou plusieurs couches formant tampon (103a-c), une couche de tissu d'espacement (104) et une couche adhésive de contact avec la peau (105) ; ladite couche de support (101) et ladite couche adhésive de contact avec la peau (105) étant disposées de manière à s'étendre au-delà du contour de ladite couche d'étalement de liquide (102), ledit tampon absorbant (103) et ladite couche de tissu d'espacement (104) pour former une section de bordure (106), ladite couche de tissu d'espacement (104) comprenant une couche supérieure (104a), une couche inférieure (104b) et une couche d'interconnexion (104c) agencée entre ladite couche supérieure (104a) et ladite couche inférieure (104b) ; ladite couche d'interconnexion (104c) comprenant une pluralité de filaments pelucheux (107) s'étendant entre ladite couche supérieure (104a) et ladite couche inférieure (104b), ladite couche supérieure (104a) de ladite couche de tissu d'espacement (104) étant définie par des bords périphériques, et au moins l'une de ladite couche d'étalement de liquide (102) et de ladite ou desdites couche(s) formant tampon (103a-c) dudit tampon absorbant (103) étant disposée de manière à recouvrir lesdits bords périphériques de ladite couche supérieure (104a) de ladite couche de tissu d'espacement (104).

2. Pansement pour thérapie par pression négative (100) selon la revendication 1, dans lequel ladite couche de support (101) comporte une section centrale et une section de bord entourant ladite section centrale, ladite section centrale a une forme préformée complémentaire de la forme d'au moins ledit tampon absorbant (103), et ladite section de bord fait partie de ladite section de bordure (106).

3. Pansement pour thérapie par pression négative (100) selon la revendication 1 ou la revendication 2, dans lequel ladite couche de tissu d'espacement (104) a une épaisseur t1, et au moins l'une de ladite couche d'étalement de liquide (102) et de ladite ou desdites couches formant tampon (103a-c) dudit tampon absorbant (103) est disposée de manière à recouvrir lesdits bords périphériques de ladite couche supérieure (104a) d'une distance dl, ladite distance d1 correspondant à 0,7 à 4,0 t1, de préférence 1,0 à 2,0 t1.

4. Pansement pour thérapie par pression négative (100) selon la revendication 1, comprenant en outre un élément de couplage (108) configuré pour connecter ledit pansement à une source de pression négative, ladite couche de support (101) et au moins une section dudit tampon absorbant (103) comprenant une ouverture (109) sous ledit élément de couplage (108) ; ladite couche d'étalement de liquide (102) étant dépourvue d'ouverture.

5. Pansement pour thérapie par pression négative (100) selon la revendication 1, dans lequel ladite couche d'étalement de liquide (102) est une couche continue s'étendant sur toute la surface dudit tampon absorbant (103).

6. Pansement pour thérapie par pression négative (100) selon l'une quelconque des revendications précédentes, dans lequel ladite couche d'étalement de liquide (102) est une couche non tissée.

7. Pansement pour thérapie par pression négative (100) selon l'une quelconque des revendications précédentes, dans lequel ledit tampon absorbant (103) comprend une première couche de distribution de liquide (103a), une couche super-absorbante (103b) et une seconde couche de distribution de liquide (103c), ladite couche super-absorbante (103b) est disposée entre lesdites première (103a) et seconde (103c) couches de distribution de liquide, et ladite première (103a) ou ladite seconde (103b) couche de distribution de liquide est une couche non tissée.

8. Pansement pour thérapie par pression négative (100) selon l'une quelconque des revendications précédentes, dans lequel à la fois ladite couche d'étalement de liquide (102) et ladite couche non tissée dudit tampon absorbant (103) sont agencées pour chevaucher lesdits bords périphériques de ladite couche supérieure (104a) de ladite couche de tissu d'espacement (104).

9. Pansement pour thérapie par pression négative (100) selon l'une quelconque des revendications précédentes, dans lequel ladite couche d'étalement de liquide (102) et chaque couche formant tampon (103a-c) dudit tampon absorbant (103) sont agencées pour chevaucher lesdits bords périphériques de ladite couche supérieure (104a) de ladite couche de tissu d'espacement (104).

10. Pansement pour thérapie par pression négative (100) selon la revendication 9, dans lequel l'épaisseur combinée de ladite couche d'étalement de liquide (102) et dudit tampon absorbant (103) est supérieure à l'épaisseur t1 de ladite couche de tissu d'espacement (104c).

11. Pansement pour thérapie par pression négative (100) selon l'une quelconque des revendications précédentes, dans lequel ledit pansement comprend une tubulure (110), une première extrémité distale dudit tube (110) étant connectée audit élément de couplage (108) et une seconde extrémité distale de ladite tubulure (110) étant configurée pour être connectée à une source de pression négative, ladite tubulure (110) comprenant un conduit de fluide (110a) configuré pour extraire le fluide dudit pansement et un conduit d'air (110b) configuré pour alimenter en air ledit conduit de fluide (110a) et/ou ledit pansement pour thérapie par pression négative (100).

12. Système de thérapie des plaies par pression négative (NPWT) (200) comprenant :
- un pansement (100) pour la thérapie des plaies par pression négative (NPWT) selon l'une quelconque des revendications 1 à 11,
- une source de dépression,
- un moyen de collecte de fluide à distance (113) relié fluidiquement à ladite source de pression négative et audit pansement (100).

13. Système de thérapie des plaies par pression négative (200) selon la revendication 12, dans lequel ledit moyen de collecte de fluide à distance (113) est une cartouche et dans lequel ladite cartouche et ladite source de pression négative sont agencées à l'intérieur du même dispositif (114).

14. Système de thérapie des plaies en par pression négative (200) selon la revendication 13, dans lequel ledit système (200) comprend des moyens pour fournir de l'air audit pansement (100) à un débit de 2 à 7 ml/min pendant le fonctionnement.

15. Procédé de fabrication d'un pansement (100) pour la thérapie des plaies par pression négative (NPWT), ledit procédé comprenant :
(a) la prévision d'une couche de support (101),
(b) la disposition d'une couche d'étalement de liquide (102) en contact avec ladite couche de support (101),
(c) la disposition d'un tampon absorbant (103) comprenant une ou plusieurs couches formant tampon (103a-c) en contact avec ladite couche d'étalement de liquide (102),
(d) la disposition d'une couche de tissu d'espacement (104) en contact avec ledit tampon absorbant (103), en ladite couche de tissu d'espacement (104) comprenant une couche supérieure (104a), une couche inférieure (104b) et une couche d'interconnexion (104c) disposée entre ladite couche supérieure (104a) et ladite couche inférieure (104b) ; ladite couche d'interconnexion (104c) comprenant une pluralité de filaments pelucheux (107) s'étendant entre ladite couche supérieure (104a) et ladite couche inférieure (104b), ladite couche supérieure (104a) de ladite couche de tissu d'espacement (104) étant définie par des bords périphériques,
(e) la disposition d'une couche adhésive de contact avec la peau (105) en contact avec ladite couche de tissu d'espacement (104), ladite couche adhésive de contact avec la peau (105) et ladite couche de support (101) étant agencées pour s'étendre au-delà du contour de ladite couche d'étalement de liquide (106), dudit tampon absorbant (103) et de ladite couche de tissu d'espacement (104) pour former une section de bordure (102), et
au moins l'une de ladite couche d'étalement de liquide et de ladite ou desdites couches formant tampon dudit tampon absorbant (103) étant disposée de manière à chevaucher lesdits bords périphériques de ladite couche supérieure (104a) de ladite couche de tissu d'espacement (104).

16. Procédé selon la revendication 15, dans lequel ladite couche de support (101) est préformée en une forme complémentaire de la forme d'au moins ledit tampon absorbant (103) avant ou simultanément à l'étape b) consistant à disposer ladite couche d'étalement de liquide en contact avec ladite couche de support (101).

17. Procédé selon la revendication 16, dans lequel ladite couche de support (101) est préformée par
(a) mise en contact de ladite couche de support (101) avec un outil de moulage ayant une forme complémentaire à celle d'au moins ledit tampon absorbant (103), et
(b) application d'un vide à ladite couche de support (101).

18. Procédé selon la revendication 17 lorsqu'elle dépend de la revendication 15, dans lequel lesdites étapes b) à d) consistant à disposer ladite couche d'étalement de liquide (102), ledit tampon absorbant (103) et ladite couche de tissu d'espacement (104) sont exécutées simultanément avec la mise en contact de ladite couche de support (101) avec un outil de moulage ayant une forme complémentaire à la forme de ladite couche d'étalement de liquide (102) et dudit tampon absorbant (103), et de préférence tout en appliquant simultanément du simultanément sous vide à ladite couche de support (101).
